# EUROPEAN PATENT SPECIFICATION

(11) **EP 1 648 380 B1**
(45) Date of publication and mention of the grant of the patent: **08.04.2015**
(21) Application number: 04777533.3
(22) Date of filing: 02.07.2004
(51) Int. Cl.: C12N 5/071, A61K 35/48

(54) **COMPOSITIONS CONTAINING SERTOLI CELLS AND MYOID CELLS AND USE THEREOF IN CELLULAR TRANSPLANTS**
ZUSAMMENSETZUNGEN MIT SERTOLI-ZELLEN UND MYOID-ZELLEN UND IHRE VERWENDUNG IN ZELLULÄREN TRANSPLANTEN
COMPOSITIONS CONTENANT DES CELLULES DE SERTOLI ET DES CELLULES MYOIDES ET LEURS UTILISATIONS DANS DES GREFFES CELLULAIRES

(30) Priority: 03.07.2003 US 484960 P
(43) Date of publication of application: 26.04.2006
(73) Proprietor: Sertoli Technologies, LLC, Tucson, AZ 85711-3335 (US)
(72) Inventor: Dufour, Jannette, Lubbock, TX 79416 (US); Halberstadt, Craig, Charlotte, NC 28270 (US); Hemendinger, Richelle, Harrisburg, NC 28075 (US); Rajotte, Ray V., Edmonton, Alberta T6W 1J6 (CA); Vasconcellos, Alfred V., Cranston, Rhode Island 02921 (US); Gores, Paul, Charlotte, NC 28277 (US); Emerich, Dwaine, Glocester, RI 02857 (US); Korbutt, Greg, St. Albert, Alberta T8N 6B8 (CA)
(74) Representative: Schlich, George
(86) International application number: PCT/US2004/021462
(87) International publication number: WO 2005/018540

(56) References cited:
- KORBUTT G S ET AL: "Cotransplantation of allogenic islets h 80 dB linearity for ISDN U-interfac with allogenic testicular cell aggregates allows long-term graft survival without systemic immunosuppression" DIABETES, AMERICAN DIABETES ASSOCIATION, US, vol. 46, 1 February 1997 (1997-02-01), pages 317-322, XP002962502 ISSN: 0012-1797
- WILLING A E ET AL: "Sertoli cells decrease microglial response and increase engraftment of human hNT neurons in the hemiparkinsonian rat striatum." BRAIN RESEARCH BULLETIN 1 MAR 1999, vol. 48, no. 4, 1 March 1999 (1999-03-01), pages 441-444, XP002549569 ISSN: 0361-9230
- PALOMBI F ET AL: "Alkaline phosphatase is a marker for myoid cells in cultures of rat peritubular and tubular tissue." BIOLOGY OF REPRODUCTION DEC 1988, vol. 39, no. 5, December 1988 (1988-12), pages 1101-1109, XP002549570 ISSN: 0006-3363
- DUFOUR ET AL.: 'Development of an in vivo model to study testicular morphogenesis' JOURNAL OF ANDROLOGY vol. 23, no. 5, 2002, pages 635 - 644, XP003000316
- DUFOUR JANNETTE M ET AL: "Comparison of successful and unsuccessful islet/sertoli cell cotransplant grafts in streptozotocin-induced diabetic mice", CELL TRANSPLANTATION, vol. 16, no. 10, 2007, pages 1029-1038, ISSN: 0963-6897
- DUFOUR JANNETTE M ET AL: "Sertoli cell line lacks the immunoprotective properties associated with primary sertoli cells", CELL TRANSPLANTATION, vol. 17, no. 5, 2008, pages 525-534, ISSN: 0963-6897
- SIPIONE S ET AL: "Identification of a Novel Human Granzyme B Inhibitor Secreted by Cultured Sertoli Cells", JOURNAL OF IMMUNOLOGY, AMERICAN ASSOCIATION OF IMMUNOLOGISTS, US, vol. 177, no. 8, 1 October 2006 (2006-10-01), pages 5051-5058, XP002564096, ISSN: 0022-1767
- SCARPINO S ET AL: "A rapid method of Sertoli cell isolation by DSA lectin, allowing mitotic analyses.", MOLECULAR AND CELLULAR ENDOCRINOLOGY 25 NOV 1998, vol. 146, no. 1-2, 25 November 1998 (1998-11-25), pages 121-127, ISSN: 0303-7207

## Description

### FIELD OF THE INVENTION

The present invention relates to Sertoli cells and myoid cells for creating an immunologically privileged site in a mammalian subject, thereby facilitating the transplantation of cells that produce a biological factor in the treatment of a disease that results from a deficiency of such biological factor. Pharmaceutical compositions containing Sertoli cells and myoid cells, as well as pharmaceutical compositions for use in therapeutic methods are provided by the present invention.

### BACKGROUND OF THE INVENTION

The testis, brain, and anterior chamber of the eye are considered immunoprivileged sites and have been investigated for their ability to protect cellular grafts [1-3]. Allogeneic and concordant xenogeneic tissues transplanted into the testis survive long term [4-10].

Moreover, parathyroid allografts in the testis restore normocalcemia in parathyroidectomized rats [8] and islets transplanted into the intra-abdominally placed testis correct hyperglycemia in diabetic rodents [9, 10]. Immune tolerance in the testis is due at least in part to Sertoli cells; because grafts are still protected after the selective destruction of the other major components of the testis, Leydig cells and germ cells [11, 12]. Furthermore, rodent Sertoli cells can survive as allografts [13, 14] and allogeneic islets or xenogeneic adrenal chromaffin cells are protected from immune-mediated rejection when co-transplanted with Sertoli cells [14-16]. Sertoli cells comprise a major component of the mammalian testis and are considered "nurse" cells because they provide numerous factors required for the orderly development and protection of spermatozoa [17]. As the germ cells mature they develop surface antigens which are recognized as foreign by the immune system making it necessary for the testis to develop a mechanism for protecting the developing germinal cells [18, 19]. It is believed that Sertoli cells protect the germ cells by creation of the blood-testis barrier [20,21] and secretion of factors that may lead to local immune tolerance [13, 22,-26]. Sertoli cells are known to produce Fas ligand (FasL) [13], transforming growth factor b (TGF β) [27], and clusterin [28], which are believed to have immunoprotective [13, 29], anti-inflammatory [30, 31], tolerogenic properties [28, 32], respectively. It is postulated that these proteins may be responsible for the immunoprotective ability of Sertoli cells. Further study of these factors in an established model of Sertoli cell transplantation may provide clues to factors necessary for creating a local tolerogenic environment.

Recently, it has been shown that an immunoprivileged site can be created by preegrafting Sertoli cells, which site subsequently protected allogeneic islets from rejection [56]. Long-term survival of porcine islets placed in the repositioned intra-abdominal testis was reported in non-immunosuppressed beagle dogs [57]. Survival of syngeneic rat islet grafts transplanted in the omental pouch was also reported [58]. A model for studying islet development and xenotransplantation has been described [59]. Recent reports have shown the survival of Sertoli cells from 12-week-old Yorkshire pigs in the rat brain, an immunoprivileged site [33]. However, survival of discordant xenogeneic porcine Sertoli cells has not been demonstrated in a non-immunoprivileged site.,

More specifically, Korbutt GS, et al [14] discloses transplantation of rat Sertoli cells and long-term graft survival without systemic immunosuppression. Scarpino S, et al, Molecular Cellular Endocrinology, 146 (1998) pp121-127 discloses isolation of rat Sertoli cells using DSA lectin, noting contamination of the preparations by other cell types, including 4% that stained for alkaline phosphatase activity and were identified as myoid cells.

### SUMMARY OF THE INVENTION

The present inventors have demonstrated a long-term survival of neonatal porcine Sertoli cells (NPSCs) in non-immunosuppressed Lewis rats when transplanted underneath the kidney capsule, a non-immunoprivileged site. The present inventors have surprisingly found that the long-term survival of Sertoli cells depends upon the presence of myoid cells.

Accordingly, the present invention provides a pharmaceutical composition comprising Sertoli cells, myoid cells and a pharmaceutically acceptable carrier, for use in creating an immunologically privileged site in a mammal, wherein the ratio of said myoid cells to said Sertoli cells is at least 5:95.

The invention also provides Sertoli cells, myoid cells and a therapeutically effective amount of cells that produce insulin, for use in a method for the treatment of diabetes mellitus, wherein the ratio of said myoid cells to said Sertoli cells is at least 5:95.

In a preferred embodiment, the ratio of myoid cells versus Sertoli cells in the pharmaceutical composition is at least 25:75. Preferably, the ratio is in the range of 5:95 to 65:35.

Sertoli cells and myoid cells each can be isolated from the testis of a mammal, preferably a pig, and more preferably a neonatal pig of 60 days old or younger, even more preferably, a neonatal pig of 5 days old or younger. Alternatively, Sertoli cells can be obtained from a cell line, which can be either an established cell line such as TM4, or a stem cell line that could be derived from human tissue.

The pharmaceutical composition of the present invention can also include cells that produce a biological factor. In a preferred embodiment, cells that produce a biological factor are cells of pancreatic islets. As to the relative amount of Sertoli cells versus cells of pancreatic islets in the pharmaceutical composition, it is preferred that not more than 2 x 10⁶ Sertoli cells, i.e. 2 x 10⁶ or fewer Sertoli cells, are used per 800 islets

In another preferred embodiment, the pharmaceutical composition is provided in a device suitable for implantation into a mammalian subject.

In another embodiment, the present invention is for use in method of creating an immunologically privileged site in a mammalian subject, preferably a human subject, by administering Sertoli cells and myoid cells into the subject.

Sertoli cells and myoid cells used in the administration can each be isolated from the testis of a mammal, preferably a pig, and more preferably a neonatal pig of 60 days old or younger, and even more preferably, a neonatal pig of 5 days old or younger. Alternatively, Sertoli cells used in the administration can be obtained from a Sertoli cell line, either an established cell line such as TM4, or a stem cell line that could be derived from human tissue. Such Sertoli cells prepared from a cell line can be admixed with myoid cells for administration to a subject.

Preferably, Sertoli cells and myoid cells are co-cultured prior to administration under conditions, e.g. for a period of about 24-48 hours, to form Sertoli-myoid cell aggregates.

Sertoli cells and myoid cells can be administered subcutaneously into a site in the subject or administered intramuscularly. Preferably, the site is selected from the brain, the renal subcapsular space, the liver subcapsular space, the hepatic portal vein, the omental pouch, or the subcutaneous fascia.

According to the present invention, the total amount of Sertoli cells and myoid cells administered can be in the range of 10⁵ to 10⁸ cells. Generally speaking, the amount of cells required to create an immunologically privileged site in a human subject is more than the amount required in a mouse, for example. For administration to a human subject, the total amount of cells administered is preferably about 10⁷ to 10⁸ cells.

The administration can be achieved by implantation of a device encapsulating the cells, or by transplantation. The transplantation can either be allograft or xenograft.

In still another embodiment, the present invention is for use in treating a disease that results from a deficiency of a biological factor in a mammalian subject, preferably a human, by administering Sertoli cells, myoid cells and a therapeutically effective amount of cells that produce said biological factor to the subject.

Sertoli cells and myoid cells administered to the subject are preferably isolated from the testis of a mammal, preferably a pig, and more preferably, a neonatal pig of 60 days old or younger, and even more preferably, a neonatal pig of 5 days old or younger. Alternatively, Sertoli cells can be obtained from a Sertoli cell line, either an established cell line such as TM4, or a stem cell line that could be derived from human tissue. Such Sertoli cells prepared from a cell line can be admixed with myoid cells and the cells that produce the desired biological factor.

In a preferred embodiment, the biological factor is a hormone.

In another preferred embodiment, the disease is diabetes mellitus and the cells that produce a biological factor are cells of pancreatic islets. It is preferred that the ratio of Sertoli cells versus cells of pancreatic islets is such that not more than 2 x 10⁶ Sertoli cells are used per 800 islets. It is also preferred that prior to administration of the cells to a subject, Sertoli cells, myoid cells and islet cells are co-cultured under conditions, e.g., for a period of about 24-48 hours, to form Sertoli-myoid-islet cell aggregates for administration.

The cells can be administered subcutaneously into a site in the subject or administered intramuscularly. Preferably, the site is selected from the brain, the renal subcapsular space, the liver subcapsular space, the hepatic portal vein, the omental pouch, or the subcutaneous fascia.

The total amount of Sertoli-cells, myoid cells and cells that produce a biological factor administered to the subject is in the range of 10⁵ to 10⁸ cells. Generally spearing, the amount of cells required to treat a human subject is more than the amount required to treat, e.g., a mouse. For administration to a human subject, the total amount of cells administered is preferably about 10⁷ to 10⁸ cells.

The administration can be achieved by implantation of a device encapsulating the cells, or by transplantation. The transplantation can be either allograft or xenograft.

### BRIEF DESCRIPTION OF THE DRAWINGS

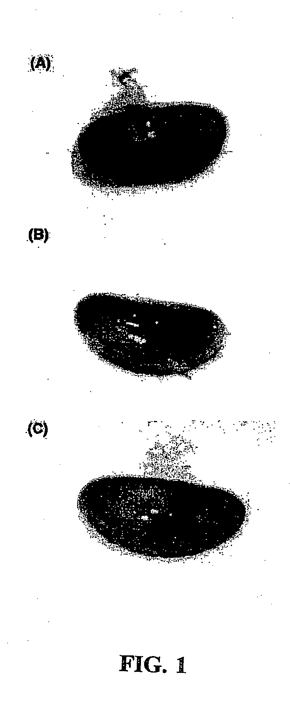

**FIGS. 1A-1C****.** Photomicrographs of NPSC grafts underneath the left kidney capsule of Lewis rats. Grafts were removed at 30 (A), 40 (B), and 60 (C) days post-transplant and photographed for macroscopic identification of transplanted tissue.

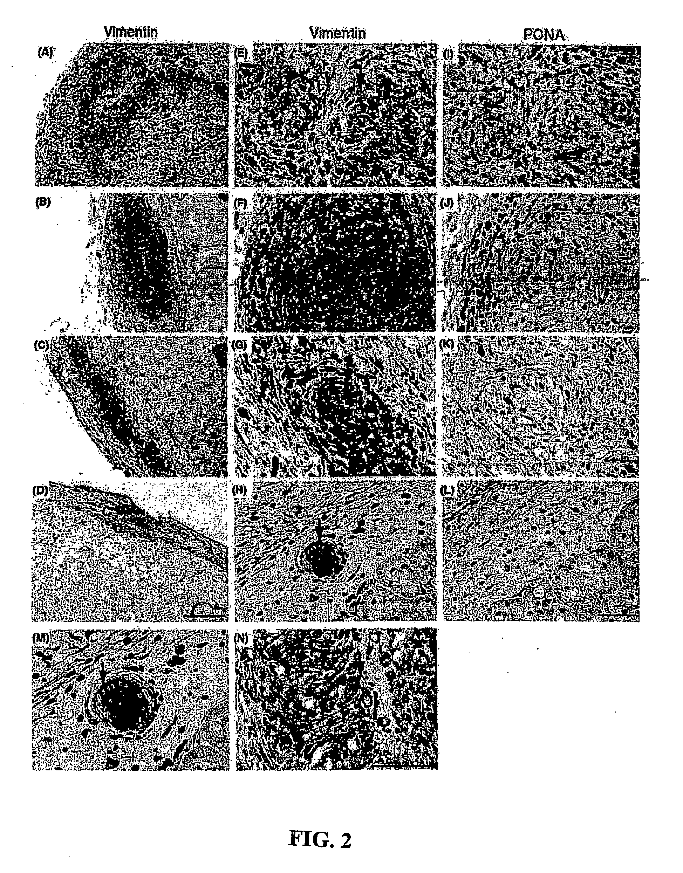

**FIGS. 2A-2N****.** Survival tubule formation, and Sertoli cell proliferation in NPSC grafts transplanted to Lewis rats. Grafts were removed at 20 (A, E, 1), 40 (B, F, J, N), 60 (C, G, K) and 90 (D, H, L, M) days post-transplant and immunostained for vimentin (AH, M, N) or PCNA (I-L). T, tubule; C, sertoli cell cluster; L lymphocytes; Arrow, Sertoli cell nucleus. Photomicrographs E-H are from serial sections to I-L, respectively. N is higher magnification of tubule shown in B, M is higher magnification of tubule shown in H. Bar in D, 200 µm for A-D. Bar in L. 50 µm for E-L. Bar in N, 50 µm for M and N.

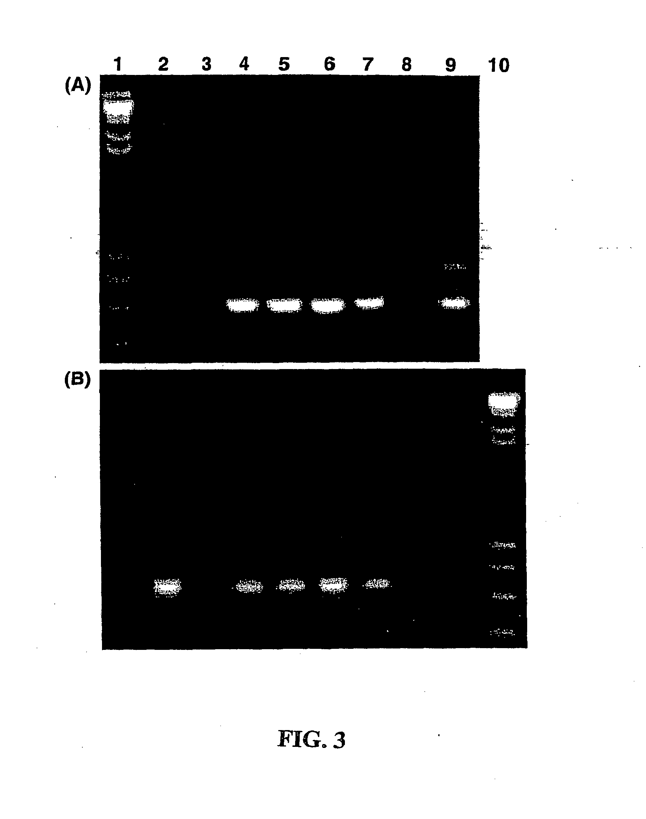

**FIGS. 3A-3B****.** Detection of COII DNA in NPSC grafts from Lewis rats to demonstrate xenogeneic survival of porcine tissue. Nested PCR was performed for porcine COII (A) while single stage PCR was performed for mouse GAPDH (B). Grafts were removed at 20 (lane 4). 30 (lane 5). 60 (lane 6) and 90 (lane 7) days post-transplant. Negative controls included DNA isolated from non-transplanted Lewis rat kidney (lane 2) while positive controls included DNA from cellular aggregates prior to transplantation (lane 9).

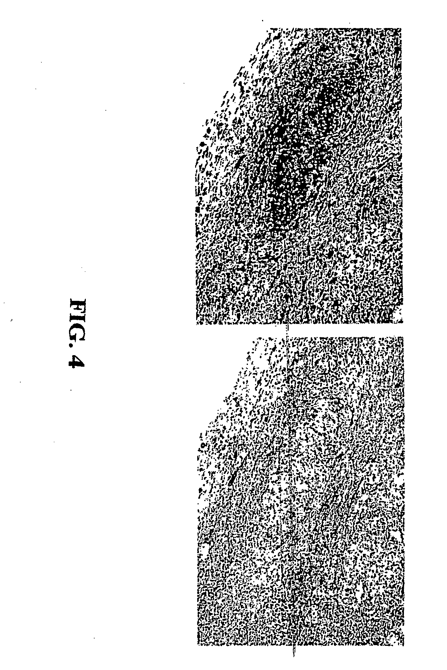

**FIG. 4****.** Photomicrographs of sections of kidney capsule following transplantation of 11 million neonatal porcine Sertoli cells underneath the kidney capsule of Lewis rats. The graft site was removed 20 days following transplant and stained for Vimentin (a marker of Sertoli cells) and smooth muscle alpha actin (a marker for myoid cells). As shown in the left panel Vimentin staining revealed the presence of viable porcine Sertoli cells. The right panel demonstrates that these graft sites also contained viable porcine myoid cells distributed throughout the graft site.

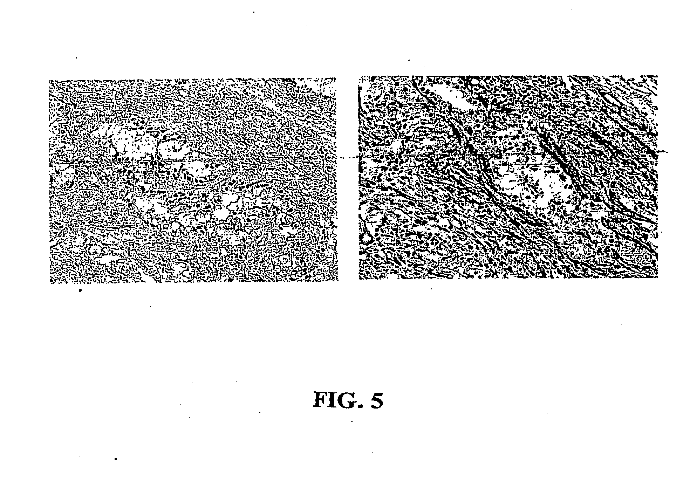

**FIG. 5****.** Photomicrographs of sections of kidney capsule following co-transplantation of 11 million Lewis Sertoli cells with 2000 Lewis islets into diabetic Wistar-Furth rats. The graft site was removed 33 days following transplant and stained for GATA-4 (a marker of Sertoli cells) and smooth muscle alpha actin (a marker for myoid cells). As shown in the left panel GATA-4 staining revealed the presence of viable Sertoli cells that were re-organized into tubule-like structures. The right panel demonstrates that these graft sites also contained viable myoid cells distributed throughout the graft site. Importantly, animals with viable grafts also became normoglycemic.

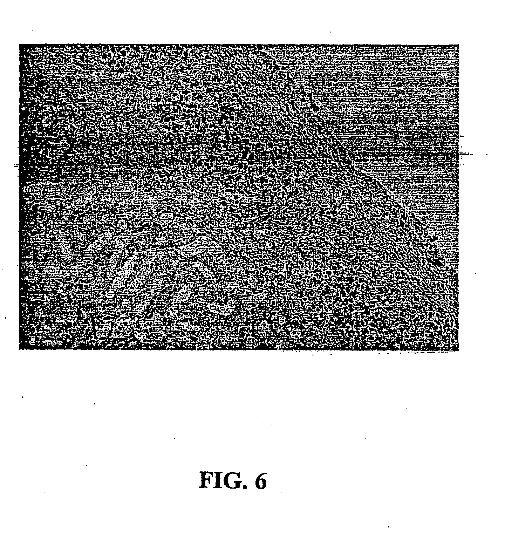

**FIG. 6****.** Photomicrograph of section of kidney capsule following co-transplantation of 4 million MSC-1 cells with 500 Balb/c islets into diabetic C3H mice. The graft site was removed 27 days following transplant and stained for T antigen to identify MSC-1 cells and insulin to identify islets. Moderate-sized pockets of viable MSC-1 were found but this Sertoli cells did not provide any immunoprotection for co-grafted islets.

### DETAILED DESCRIPTION OF THE INVENTION

### EXAMPLE 1

### Materials and methods

### Animals

Male Landrace-Yorkshire neonatal pigs (aged 1 to 3 days) were used as Sertoli cell donors. Male Lewis rats (RT1^{1/1}, Charles River Canada, St Constant, Quebec, Canada), aged 8 to 10 weeks, were used as recipients.

### Sertoli cell isolation

The NPSCs were isolated using a technique similar to that previously described for rat Sertoli cells [14]. Briefly, 1 to 3-day old-male Landrace-Yorkshire neonatal pigs were anesthetized with Halothane, testicles were surgically removed and placed in 50-ml conical tubes containing cold (4°C) Hank's balanced salt solution (HBSS) supplemented with 0.25% (w/v) bovine serum albumin (fraction V; Sigma Chemical Co., St Louis, MO, USA). The testes were cut into 1-mm fragments with scissors, digested for 10 min at 37°C with collagenase (2.5 mg/ml; Sigma Type V, St Louis, MO, USA) and then washed three times with HBSS. The tissue was resuspended in calcium-free medium supplemented with 1 mM EGTA and further digested with trypsin (25 µg/ml; Boehringer Mannheim, Laval, Canada) and DNase (4 µg/ml, Boehringer) for 10 min at 37°C. The digest was passed through a 500-µm nylon mesh, washed with HBSS and cultured in non-treated Petri dishes (15 cm diameter) containing 60 to 80 x 10⁶ cells and 35 ml of Ham's F10 media supplemented with 10 mmol/l D-glucose, 2 mmol/l 1-glutamine, 50 µmol/l isobutylmethylxanthine, 0.5% bovine serum albumin, 10 mmol/l nicotinamide, 100 U/ml penicillin, 100 µg/ml streptomycin, and 10% heat-inactivated neonatal porcine serum. Cells were incubated for 48 h at 37°C to allow the formation of cellular aggregates (100 to 300 µm diameter).

### Characterization and transplantation of Sertoli cell grafts

After culture and immediately prior to transplantation, the purity, viability and mass of NPSCs was determined on the basis of the proportion of vimentin-positive Sertoli cells, smooth muscle alpha actin-positive peritubular myoid cells, trypan blue dye exclusion and DNA content, respectively. As it is difficult to accurately count cells in a three-dimensional structure, we assessed the number of vimentin-positive Sertoli cells and smooth muscle alpha actin-positive peritubular myoid cells in representative aliquots after dissociation of the aggregates into single cells using techniques previously described for islet dissociation [34]. The dispersed cell suspension was allowed to attach to Histobond adhesive microscope slides (F.G.R. Steinmetz Inc., Surry, BC, Canada), fixed with Bouin's solution for 30 min, washed with 70% ethanol and immunostained using the Sertoli cell marker, vimentin [14] or the myoid cell marker, smooth muscle alpha actin [35]. In each preparation a minimum of 500 single cells were counted.

Cell viability was measured at the time of transplantation using a trypan blue dye exclusion assay. This assay is based on the principle that viable cells do not take up trypan blue dye while non-viable cells will take up the dye. Cellular aggregates were dissociated as described previously [34] and incubated with 0.1 % trypan blue dye (Sigma) for 5 min. Stained and nonstained cells were counted and the percentage of viable cells calculated.

To assess cell recovery after culture and standardize the mass of NPSCs transplanted in each experiment, three representative aliquots of the cellular aggregates were measured for total cellular DNA content using a Hoefer DyNa Quant 200 fluormetric assay (Amersham Pharmacia Biotech, San Francisco, CA, USA). Aliquots were washed with citrate buffer [150 mmol/l NaCl, 15 mmol/l citrate, 3 mmol/l ethylene diamine tetraacetic acid (EDTA), pH 7.4], resuspended in TNE buffer (10 mM Tris, 0.2 mM NaCl, 1 mM EDTA, pH 7.4) and solicited. Aliquots of 10 µl were assayed in triplicate by diluting them in 2 ml of assay solution (0.1 µg/ml Hoechst 33258 in 1 X TNE) and measuring fluorescence (365 nm excitation/460 nm emission). Samples were run in parallel with and diluted in proportion to a six-point (0-500 ng/ml) DNA standard curve generated using calf thymus DNA. When considering the mean DNA recovery in each preparation, with the DNA content of porcine Sertoli cells (6.6 pg DNA/cell), the total number of cells was subsequently calculated. For transplantation, aliquots consisting of 11 x 10⁶ cells were placed in polypropylene microcentrifuge tubes, aspirated into polyethylene tubing (PE-50), pelleted by centrifugation, and gently placed under the left renal subcapsular space of Halothane-anesthetized Lewis rats [14].

### Assessment of NPSC survival post-transplantation

### Histology

Nephrectomies were performed for morphological analysis at 4 (n = 3), 20 (n = 5), 30 (n = 8), 40 (n = 5), 60 (n = 10) and 90 (n = 3) days posttransplant. The graft-bearing kidneys were immersed in Z-fix and embedded in paraffin. After deparaffinization and rehydration, tissue sections were immunostained using antigen retrieval by heating for 15 min in 0.01 M sodium citrate buffer (pH 6.0), in a microwave at full power [14, 36, 37]. Consecutive sections were incubated with 10% hydrogen peroxide to quench endogenous peroxidases, blocked with non-specific serum, and incubated with either mouse monoclonal antivimentin (PCNA; 1:100; Dako, Carpinteria, CA, USA), or mouse monoclonal anti-proliferating cell nuclear antigen (1 : 50; Dako) for 30 min. Sections were then incubated with biotinylated goat anti-mouse secondary antibody (1:200; Vector Laboratories, Burlingame, CA, USA) for 20 min followed by peroxidase-streptavidin, substratechromagen (aminoethyl carbazole) and then stained with hematoxylin (Zymed Laboratories Inc., San Francisco, CA, USA). Positive controls included sections of neonatal pig testes, whereas negative controls included omission of primary antibody. Positive controls demonstrated vimentin and PCNA immunoreactivity within Sertoli cells and negative controls had no staining.

### DNA extraction. PCR and sequence analysis

Nephrectomies were also performed for DNA extraction, under sterile conditions to prevent cross contamination, at 4, 20, 30, 40, 60, and 90 days post-transplant (n ≥ 3/timepoint). Tissue from grafts and non-graft-bearing kidneys were immediately frozen and stored at - 80°C for later DNA isolation. After thawing on ice, samples were resuspended in 350 µl of lysis buffer [50 mM Tris, 100 mM EDTA, 400 mM NaCl, 0.5% sodium dodecyl sulfate (SDS)] and treated with 0.2 mg/ml proteinase K (Sigma) overnight at 55°C. Following proteinase K inactivation, chloroform extraction and ethanol precipitation, DNA pellets were washed in 70% ethanol, air dried and dissolved in 100 µl of DNase/RNase free water (Sigma). DNA concentration and quality were determined by spectrophotometric analysis prior to amplification.

To confirm the presence of porcine DNA, primers specific to the porcine mitochondrial gene encoding COII [38] and to the housekeeping gene, mouse glyceraldehyde-3-phosphate dehydrogenase (GAPDH) were used. The COII PCR was two stage (nested) while GAPDH was single stage. The starting template in the first amplification of the nested and single stage PCR was 0.5 to 1 µg of DNA in a 50-µl volume containing1X PCR buffer, 2 mM MgCl₂, 300 nM of each primer, 200 µM dNTP and 1.5 U Taq DNA Polymerase (Invitrogen Carlsbad, CA, USA). In the nested PCR, 2 to 3% of the first reaction served as the template in the second round of amplification. All amplifications were performed using the Gene Amp PCR System 9700 (Applied Biosystems, Foster City, CA, USA) with the following cycling conditions: 94°C 3-min denaturation followed by either 25 cycles (first PCR) or 30 cycles (second PCR) of 94°C for 30 s, 56°C for 30 s, 72°C for 30 s, and final extension of 72°C for 10 min. PCR products were electrophoresed through an ethidium bromide stained agarose gel (1.5%) and photographed. COII PCR products of the expected size were ligated into the pCR4-TOPO vector (TOPO TA Cloning Kit for Sequencing, Invitrogen) and sequenced. Unknown sequences were analyzed using BLAST (NCBI) and compared with known GenBank sequences. The sequenced band was identical to the region of porcine COII DNA from 7112 to 7426 bp (GENBANK accession number AF304202) verifying the specificity of the PCR product. Primers for COII were: first PCR FORGCT TAC CCT TTC CAA CTA GGC TTC and REV- TTC GAA GTA CTT TAA TGG GAC AAG, second PCR FOR- CAC ACA CTA GCA CAA TGG ATG CC and REV- GAG GAT ACT AAT ATT CGG ATT GTT AT. Primers for GAPDH were: FOR- AAT CCC ATC ACC ATC TTC CA and REV- GGC AGT GAT GGC ATG GAC TG.

### Results

### NPSC aggregate characterization

Porcine Sertoli cells were isolated from neonatal testes and cultured for 2 days to allow for formation of cellular aggregates. Prior to transplantation, the composition of these cellular aggregates was determined by assessing the proportion of immunoreactive vimentin-positive Sertoli cells and smooth muscle alpha actin positive myoid cells [14, 35]. From a total of four independent preparations, these aggregates were shown to contain 92.2 ± 5.1% Sertoli cells and 2.2 ± 0.7% myoid cells. The remaining cell population (i.e. <6%) is likely composed of germ cells, Leydig cells and fibroblasts.

On the basis of the total DNA content of each NPSC preparation and the observation that single NPSCs contain 6.6 pg DNA/cell (data not shown), it was calculated that 52.5 ± 13.3 x·10⁶ cells were obtained per testis and each implant contained 11 x 10⁶ cells. In view of the percentage of vimentin-positive Sertoli cells, the number of Sertoli cells within each graft should therefore be approximately 10.1 x 10⁶. In addition, the cell viability was measured by trypan blue dye exclusion and the cellular preparation at the time of transplantation was shown to consist of 98.6 ± 1.7% viable cells.

### Survival of porcine Sertoli cells

The NPSCs were implanted under the kidney capsule of immunocompetent Lewis rats to ascertain whether they could survive as discordant xenografts. Macroscopically at 4, 20, 30, 40, 60 and 90 days post-transplant, Sertoli cell grafts were easily identifiable with extensive neovascularization (Fig. 1). When tissue sections were examined histologically, NPSCs were identified in grafts at all time points with 66 to 100% of the grafts containing Sertoli cells (Table 1). The vimentin-positive Sertoli cells were predominately arranged as either clusters of cells or tubule-like structures (Fig. 2A-D). When the Sertoli cells were organized in clusters, they were aggregated in large swirling circular clumps, similar to that of seminiferous cords present in the embryonic testis during cord formation (Fig. 2E-G) [39]. When arranged in tubule-like structures, most of the Sertoli cells were not aligned with their nuclei along the basal edge of the tubule but were instead localized throughout the epithelial layer (Fig. 2N). However, occasionally tubules were observed with the Sertoli cell nuclei arranged as in the native testis (Fig. 2M).

To further confirm the survival of Sertoli cells in the xenografts, PCR for a porcine-specific gene was performed. After 4, 20, 30, 40, 60 and 90 days posttransplant grafts were removed and the DNA extracted for porcine mitochondrial COII PCR. COII is a marker for porcine tissue [38] and was used in the present study to confirm the presence of NPSCs in the recipients. Negative controls consisting of rat tissue from the untransplanted kidney were performed for each graft to verify the specificity of the primers for porcine DNA. No positive signal was detected in the non-transplanted kidneys (Fig. 3, lane 2), while COII was detected at the expected size of 320 bp in grafts at all time points analyzed (Fig. 3, lanes 4-7). Furthermore, the 320 bp band was sequenced and found to be identical to the region of porcine COII DNA from 7112 to 7426 bp (GENBANK accession number AF304202), verifying the specificity of the PCR product. By PCR 56 to 100% of the grafts were positive for porcine tissue (Table 1), thereby demonstrating that NPSCs were able to survive at least 90 days post-transplant in Lewis rats.

**Table 1**

| Percentage survival of NPSC xenografts transplanted to Lewis rats as measured by vimentin immunohistochemistry and COII PCR | | | | | | |
|---|---|---|---|---|---|---|
| Days Post-transplant | 4 (n=3^{a}) | 20 (n= 8) | 30 (n= 11) | 40 (n = 8) | 60 (n= 19) | 90 (n= 3^{a}) |
| Vimentin | 100 (3/3) | 100 (5/5) | 88 (7/8) | 100 (5/5) | 100 (10/10) | 66 (2/3) |
| COII | 100 (3/3) | 100 (3/3) | 88 (2/3) | 100 (3/3) | 56 (5/9) | 66 (2/3) |

| | | | | | | |
|---|---|---|---|---|---|---|
| ^{a}Grafts were used for both immunostaining and PCR. | | | | | | |

Macroscopically, extensive growth of the Sertoli cell grafts was observed at 20 days post-transplant. Due to the apparent growth of the grafts as well as the potential for unregulated proliferation of the grafted cells, tissue sections were immunostained for PCNA to identify dividing cells. PCNA is involved in DNA replication and is localized to proliferating cells [40-42]. When consecutive sections were immunostained for vimentin (Fig. 2E-H) and PCNA(Fig. 2I-L), some PCNA positive cells were detected in grafts harvested at 20 and 30 days post-transplant (Fig. 2I, data not shown); however, by 40 days, most Sertoli cells were no longer dividing (Fig. 2J). The number of proliferating Sertoli cells continued to decrease as the time post-transplant increased with very few proliferating Sertoli cells at 60 and 90 days post-transplant (Figs. 2K, L). This suggests that the Sertoli cells cease to divide by 60 days post-transplant and therefore decreases the chance of forming tumors.

### Discussion

Our results indicate that NPSCs survive long term following xenogeneic transplantation in non-immunosuppressed Lewis rats. Survival was determined histologically by vimentin immunostaining and further verified by COII PCR. Although other studies have reported survival of discordant [pig-to-rat; 33] and concordant [rat-to mouse; 43] Sertoli cells transplanted in rodents, these grafts were either implanted in the brain [33] which is considered an immunoprivileged site [3], or they were placed in an immunoprotective alginate microcapsule [43]. To our knowledge, the present study is the first to report survival of a discordant xenograft without using immunosuppression or any other immune-modulating intervention. The ability of NPSCs to survive in xenogeneic recipients indicates that they likely synthesize and secrete immune-modulating factor(s) that prevent their rejection. Further study of these factors may provide a model to examine the immunology of tolerance. For example, Sertoli cells are known to produce FasL [13], TGF β [27] and clusterin [28] which are all suggested to play a role in graft protection. Sertoli cells also secrete unidentified factors that decrease IL-2 production [24] and T cell proliferation [24-26]. Previous data indicate that FasL secreted by Sertoli cells may play a role in the survival of mouse testicular tissue fragments transplanted under the kidney capsule of allogeneic recipients [13]. In particular, testicular tissue isolated from *gld* mice (lacking functional FasL) transplanted as allografts were no longer present after 7 days while grafts from wild type mice (producing functional FasL) survived for 28 days [13]. However, more recent papers suggest that the immunoprotective effect Sertoli cells exhibited when co-transplanted with non-obese diabetic (NOD) mouse islets in diabetic NOD mice is not associated with FasL [22, 23], but that FasL is rather detrimental and correlates with neutrophil recruitment and subsequent graft destruction [22]. On the other hand, our previous study using the NOD mouse Sertoli cell/islet co-transplant model indicates that TGF β plays a protective role in preventing islet destruction [23].

It is likely that a combination of immunomodulating factors, as opposed to a single protein produced by NPSCs, permits their survival in xenogeneic recipients. One example is a report by Chen et al. [44] demonstrating that a colon carcinoma cell line transfected to express FasL and injected subcutaneously was rapidly rejected by neutrophil recruitment and activation [44]. However, when these cells were engineered to express both FasL and TGF β the grafts survived [44]. The authors suggest the combined protection is most likely due to the inhibition of p38 mitogen-activated protein kinase (MAPK) function by TGF β preventing the FasL-induced neutrophil cytotoxicity that is dependent on p38 MAPK [44]. Thus, TGF β and FasL may act synergistically to promote NPSC survival by reducing inflammation and increasing clonal deletion of lymphocytes. Clusterin, an amphipathic glycoprotein and one of the most abundant proteins secreted by Sertoli cells, is also known to have many immune-modulating functions. In particular, clusterin has been shown to: exhibit anti-inflammatory properties [28], be up-regulated and provide a local protective effect for undamaged cells after cell injury or death [28], play a role in tolerance induction for rat liver allografts [45], and inhibit activation of the complement cascade [28, 32]. As hyperacute rejection of discordant xenografts requires activation of the complement system [46], preventing this destructive process would clearly be an important mechanism to prevent rejection. It is therefore possible that TGF β and clusterin, and potentially FasL, are some of the factors secreted by NPSCs that allow them to survive xenogeneic transplantation. Further study of the mechanism for xenogeneic survival of porcine Sertoli cells may provide information on the induction of tolerance and inhibition of complement activation.

The survival of xenogeneic porcine Sertoli cells also has potential clinical applications in co-transplantation of cellular grafts for genetic engineering. The recent success of clinical islet transplantation using immunosuppressive therapy [47] provides the basis for further islet transplantation in humans. In order for this approach to become a reality for young juvenile type 1 diabetic patients, the immunosuppressive regimen would have to be replaced with an alternative strategy. Due to the ability of Sertoli cells to immunoprotect allogeneic [14,15] and NOD mouse [22,23] islet grafts in rodents, the creation of an immunoprivileged site with Sertoli cells may be a solution. However, humans are not a practical source of Sertoli cells due to the lack of available human donor tissue and data in a recent paper indicating the inability of human testicular cells to survive transplantation in mice [48]. Pigs are relatively abundant and we have shown that NPSCs are easily isolated and survive as xenografts in rodents. This implies that pigs are an ideal source of Sertoli cells for creation of an immunoprivileged site for islets. In addition to islet grafts, creation of an immunoprivileged ectopic site with NPSCs may also be useful for cellular grafts such as neuronal cells [16,33]. Otherwise, Sertoli cells could be engineered to produce therapeutic proteins such as dopamine or factor VIII to potentially treat diseases such as Parkinsonism or hemophilia.

Prior to the use of porcine Sertoli cells in a clinical situation the potential for tumor formation must be addressed. Therefore, we examined the proliferation of the NPSCs after transplantation into Lewis rats by immunostaining for PCNA and found little if any proliferation in the long-term grafts. Many PCNA-positive cells were detected at 20 days, however, this number decreased by 40 days and almost no positive cells were present at 60 and 90 days. Sertoli cells in the native testis have been shown to proliferate during post-natal testicular development until puberty at which time they cease to divide [49-51]. This growth is regulated by many factors including follicle-stimulating hormone, epidermal growth factor, nerve growth factor, neurotropin-3, and transforming growth factor-α [52-55]. Most likely the Sertoli cells at the time of transplantation to 20 days are relatively immature and so proliferate. As the time post-transplant increases the Sertoli cells likely mature and therefore the number of dividing cells decreases. This suggests that cell division of the transplanted Sertoli cells may be controlled, thereby decreasing the chances of forming tumors.

In conclusion, we have demonstrated long-term survival of xenogeneic NPSCs in rodents without immunosuppression. The survival was verified by mmunohistochemistry and PCR which suggests that Sertoli cells cannot only survive as allografts but also as discordant xenografts. Further study of this model may provide clues to the mechanism of xenograft survival and immune tolerance.

### EXAMPLE 2

### Potential Involvement Of Myoid Cells In The Local Immunoprotection Conferred By Sertoli Cell-Enriched Transplants

Sertoli cells are normal constituents of the testes where they nurse and immunologically protect the developing germ cells. Isolated Sertoli cells can ectopically create an immunoprivileged site enabling the survival of co-transplanted allogeneic or xenogeneic cells by secreting potent immunosuppressive and survival-enhancing molecules. By harnessing the natural functions of Sertoli cells it may be possible to overcome the major limitations associated with cell transplantation: the shortage of suitable donor tissue and the need for life-long immunosuppression. In pre-clinical animal models, isolated Sertoli cells (1) engraft and self-protect when transplanted into allogeneic and xenogeneic environments, (2) protect co-grafted allogeneic and xenogeneic cells from immune destruction, and (3) enable long term survival of islet co-grafts and reversal of hyperglycemia in animals with diabetes due to pancreatectomy or to autoimmune disease (non obese diabetic model).

Recent efforts to more fully characterize the cellular composition of the Sertoli cell-enriched grafts prior to transplant have revealed that a percentage of myoid cells are also contained within the preparation of successful Sertoli cell transplants. This observation is important because it suggests that the inclusion of myoid cells might play an important role in optimizing the grafts ability to confer the local immunoprotection seen in transplant studies performed by STI. There are several lines of evidence suggesting that myoid cells and Sertoli cells do indeed interact in several important ways. First, in the native testes, myoid cells are found in close proximity to Sertoli cells where they form part of the basement of the seminiferous tubule and provide a major role in the movement of intratubular fluid and the propulsion of released spermatozoa. Secondly, myoid cells play a role in regulating the function and activity of Sertoli cells by producing factors (such as PmodS) that modulate the cytodifferentiation and function of the Sertoli cell. Finally, myoid cells themselves produce growth factors and cytokines such as TGF that are believed to be immunoprotective and could add to the already potent cocktail of growth factors and cytokines produced by Sertoli cells.

The contribution of myoid cells to the immunoprotection produced by Sertoli cell-enriched transplants can be tested by the experiments as listed below:
(1) Experiments were designed to determine whether xenogeneic neonatal porcine Sertoli cells survive transplantation in rats without the use of immunosuppression. Sertoli cells were isolated, cultured and then transplanted under the kidney capsule of non-immunosuppressed Lewis rats. Using immunocytochemical techniques, the cultured cell preparations were found to contain 92+/- 5.1% Sertoli cells (Vimentin staining) and 2.2 +/- 0.7% myoid cells (smooth muscle alpha actin staining). To assess survival, grafts were removed after 4, 20, 30, 40, 60, and 90 days post-transplant and immunostained for the Sertoli cell marker vimentin. Survival was confirmed by PCR for the porcine mitochondrial cytochrome oxidase II subunit gene (COII), a marker for porcine tissue. By both methods, Sertoli cells were detected in the grafts for at least 90 days. Histologically, Sertoli cells were clustered in small aggregates or organized in tubule-like structures. Staining of adjacent sections also revealed that viable myoid cells were present within the Sertoli cell transplant sites (Figure 4). These data demonstrate that porcine Sertoli cell preparations that contain a small percentage of myoid cells survive long-term following xenotransplantation in rats.
(2) Histological studies confirm that viable co-grafted Sertoli cells and islet transplants contain abundant surviving myoid cells as well. Eleven million Lewis Sertoli cells and 2000 Lewis islets were transplanted underneath the kidney capsule of diabetic Wistar-Furth rats. The grafts were removed one month later and stained immunocytochemically for Sertoli cells using GATA-4 and myoid cells using smooth muscle alpha actin. Viable Sertoli cells were found throughout the transplant site and frequently re-organized in tubule-like structures. Using adjacent tissue sections it was found that the re-formed tubular structures were lined by abundant viable myoid cells in a manner reminiscent of the native testes (Figure 5). Together with the above studies these data suggest that the same myoid cells that are originally contained in the pre-graft preparations survive and take part in the structural re-formation of tubular structures post engraftment.
(3) One additional way of determining the relative contribution of myoid cells to the local immunoprotection described above is to transplant pure Sertoli cells without any myoid cells. Sertoli cell lines, by definition, do not contain myoid cells. By transplanting such a cell line together with islet cells it is possible to gain further insight into the contributory role of other cells including myoid cells in our earlier studies. Toward this end, 500 Balb/c islets were transplanted together with varying numbers of the cell line MSC-1 (a mouse-derived Sertoli cells line) under the kidney capsule of diabetic C3H mice. Histological analysis using T-antigen staining revealed that even though the MSC-1 cells are a tumorigenic cell line they survived poorly and were distributed in small clusters one month after transplantation. Staining of the same or adjacent sections for insulin revealed that the MSC-1 cells did not protect the co-grafted islets (Figure 6). Similarly, the inclusion of islets (500) with MSC-1 cells (2-4 million) did not produce any lasting reversal of hyperglycemia. Control animals receiving syngeneic islet grafts remained normoglycemic for 60 days (the duration of the study). However, even though co-grafted allo islets and allo MSC-1 cells produced a rapid normoglycemia, this effect was short-lived (<30 days in all cases) even in those animals receiving 4 million MSC-1 cells.

The above experiments demonstrate the presence of myoid cells in successful immunomodulating Sertoli cell rich grafts. A percentage of myoid cells between 0.5% and 65% appears to be found in successful grafts. In the next months we will attempt to identify a method to ablate the myoid cells in Sertoli cell rich grafts and to selectively vary the percentage of myoid cells in the Sertoli cell rich transplants to determine if there is a dose effect associated with survival of the graft in a foreign recipient. The Sertoli cell line data suggest that one of the reasons the MSC-1 grafts failed prematurely is the absence of a supporting population of myoid cells. To determine that the premature destruction of the graft is not due to a reduction in the cell's immunomodulating ability caused by the immortalization or proliferation process, in the next months we will attempt to transplant MSC-1 grafts with varying levels of murine myoid cells and evaluate the survival against the current baseline data.

### References

1. Streilein JW. Unraveling immune privilege. Science 1995; 270: 1158.
2. Selawry HP. Islet transplantation to immunoprivileged Sites. In: Lanza, RP, Chick, WL, eds. Pancreatic islet transplantation, Vol. II Immunomodulation of pancreatic islets. XXXX: R. G. Landes Company, 1994: 75.
3. Barker CF, Billingham RE. Immunologically privileged sites. Adv Immunol 1977; 25:1.
4. Ferguson J, Scothorne RJ. Extended survival of pancreatic islet allografts in the testis of guinea-pigs. J Anat 1977; 124: 1.
5. Bobzien B, Yasunami Y, Majercik M, Lacy PE, Davie JM. Intratesticular transplants of islet xenografts (rat to mouse). Diabetes 1983; 32: 213.
6. Bellgrau D, Selawry HP. Cyclosporine-induced tolerance to intratesticular islet xenografts. Transplantation 1990; 50: 654.
7. Whitmore WF, Gittes RF. Studies on the prostate and testis as immunologically privileged sites. Cancer Treat Rep 1977; 61: 217.
8. Naji A, Barker CF. The influence of histocompatibility and transplant site on parathyroid allograft survival. J Surg Res 1976; 20: 261.
9. Selawry HP, Whittington KB, Bellgrau D. Abdominal intratesticular islet-xenograft survival in rats. Diabetes 1989; 38 (Suppl. 1): 220.
10. Selawry HP, Whittington K. Extended allograft survival of islets grafted into intra-abdominally placed testis. Diabetes 1984; 33: 405.
11. Cameron DF, Whittington K, Schultz RE, Selawry HP. Successful islet/abdominal testis transplantation does not require Leydig cells. Transplantation 1990; 50: 649.
12. Whitmore WF III, Karsh L, Gittes RF. The role of germinal epithelium and spermatogenesis in the privileged survival of intratesticular grafts. J Urol 1985; 134: 782.
13. Bellgrau D, Gold D, Selawry H, et al. Arole for CD95 ligand in preventing graft rejection. Nature 1995; 377: 630.
14. Korbutt GS, Elliott JF, Rajotte RV. Cotransplantation of allogeneic islets with allogeneic testicular cell aggregates allows long-term graft survival without systemic immunosuppression. Diabetes 1997; 46: 317.
15. Selawry HP, Cameron DF. Sertoli cell-enriched fractions in successful islet cell transplantation. Cell Transplant 1993; 2: 123.
16. Sanberg PR, Borlongan CV, Saporta S, Cameron DF. Testis-derived Sertoli cells survive and provide localized immunoprotection for xenografts in rat brain. Nat Biotechnol 1996; 14: 1692.
17. Griswold MD. The central role of Sertoli cells in spermatogenesis. Semin Cell Dev Biol 1998; 9: 411.
18. O'Rand MG, Romrell LJ. Appearance of cell surface auto- and isoantigens during spermatogenesis in the rabbit. Dev Biol 1977; 55: 347.
19. Tung PS, Fritz IB. Specific surface antigens on rat pachytene spermatocytes and successive classes of germinal cells. Dev Biol 1978; 64: 297.
20. Dym M, Fawcett DW. The blood-testis barrier in the rat and the physiological compartmentation of the seminiferous epithelium. Biol Reprod 1970; 3: 308.
21. Russell L. Movement of spermatocytes from the basal to the adluminal compartment of the rat testis. Am J Anat 1977; 148: 313.
22. Korbutt GS, Suarez-Pinzon WL, Power RF, Rajotte RV, Rabinovitch A. Testicular Sertoli cells exert both protective and destructive effects on syngeneic islet grafts in non-obese diabetic mice. Diabetologia 2000; 43: 474.
23. Suarez-Pinzon W, Korbutt GS, Power R, et al. Testicular Sertoli cells protect islet b-cells from autoimmune destruction in NOD mice by a transforming growth factorb1-dependent mechanism. Diabetes 2000; 49: 1810.
24. Selawry HP, Kotb M, Herrod HG, Lu Z-N. Production of a factor, or factors, suppressing IL-2 production and T cell proliferation by Sertoli cell-enriched preparations. Transplantation 1991; 52: 846.
25. De Cesaris P, Filippini A, Cervelli C, et al. Immunosuppressive molecules produced by Sertoli cells cultured in vitro: biological effects on lymphocytes. Biochem Biophys Res Comm 1992; 186: 1639.
26. Wyatt CR, Law L, Magnuson JA, Griswold MD, Magnuson NS. Suppression of lymphocyte proliferation by proteins secreted by cultured Sertoli cells. J Reprod Immunol 1988; 14: 27.
27. Cupp AS, Kim G, Skinner MK. Expression and action of transforming growth factor beta (TGFβ1, TGFβ2, and TGFβ3) during embryonic rat testis development. Biol Reprod 1999; 60: 1304.
28. Bailey R, Griswold MD. Clusterin in the male reproductive system: localization and possible function. Mol Cell Endocrinol 1999; 151:17.
29. Grith TS, Brunner T, Fletcher SM, Green DR, Ferguson TA. Fas ligand-induced apoptosis as a mechanism of immune privilege. Science 1995; 270: 1189.
30. Merly F, Huard C, Asselin I, Robbins PD, Tremblay JP. Anti-inflammatory effect of transforming growth factor-β1 in myoblast transplantation. Transplantation 1998; 65: 793.
31. Wahl SM, McCartney-Francis N, Mergenhagen SE. Inflammatory and immunomodulatory roles of TGF-beta. Immunol Today 1989; 10: 258.
32. Jenne DE, Tschopp J. Molecular structure and functional characterization of a human complement cytolysis inhibitor found in blood and seminal plasma: Identity to sulfated glycoprotein 2, a constituent of rat testis fluid. Proc Natl Acad Sci USA 1989; 86: 7123.
33. Saporta S, Cameron DF, Borlongan CV, Sanberg PR. Survival of rat and porcine Sertoli cell transplants in the rat striatum without cyclosporine-Aimmunosuppression. Exp Neurol 1997; 146: 299.
34. Korbutt GS, Elliot IF, Ao Z, et al. Large scale isolation, growth, and function of porcine neonatal islet cells. J Clin Invest 1996; 97: 2119.
35. Tung PS, Fritz IB. Characterization of rat testicular peritubular myoid cells in culture: alpha-smooth muscle isoactin is a specific differentiation marker. Biol Reprod 1990; 42: 351.
36. Akmal KM, Dufour JM, Vo M, Higginson S, Kim KH. Ligand-dependent regulation of retinoic acid receptor in rat testis: In vivo response to depletion and repletion of vitamin A. Endocrinology 1998; 139: 1239.
37. Dufour JM, Kim KH. Cellular and subcellular localization of six retinoid receptors in rat testis during postnatal development: identification of potential heterodimeric receptors. Biol Reprod 1999; 61: 1300.
38. Deng Y, Tuch BE, Rawlinson WD. Transmission of porcine endogenous retroviruses in sever combined immunodeficient mice xenotransplanted with fetal porcine pancreatic cells. Transplantation 2000; 70: 1010.
39. Jost A, Magre S, Agelopoulou R. Early stages of testicular differentiation in the rat. Hum Genet 1981; 58: 59.
40. Bravo R, Frank R, Blundell PA, Macdonald-Bravo H. Cyclin/PCNA is the auxiliary protein of DNA polymerase-δ. Nature 1987; 326: 515.
41. Hall PA, Levison DA, Woods AL, et al. Proliferating cell nuclear antigen (PCNA) immunolocalization in paraffin sections: an index of cell proliferation with evidence of deregulated expression in some neoplasms. J Pathol 1990; 162: 285.
42. Prelich G, Tan CK, Kostura M, et al. Functional identity of proliferating cell nuclear antigen and a DNA polymerase-δ auxiliary protein. Nature 1987; 326: 517.
43. Yang H, Wright JR Jr. Co-encapsulation of Sertoli enriched testicular cell fractions further prolongs fish-to-mouse islet xenograft survival. Transplantation 1999; 67: 815.
44. Chen J-J, Sun Y, Nabel GJ. Regulation of the proinflammatory effects of Fas ligand (CD95L). Science 1998; 282: 1714.
45. Chiang KC, Goto S, Chen CL, et al. Clusterin may be involved in rat liver allograft tolerance. Transpl Immunol 2000; 8: 95.
46. Platt JL. A primer of xenotransplantation. In: Platt JL, ed. Xenotransplantation. XXXXX: ASM Press, 2001: 3.
47. Shapiro AMJ, Lakey JRT, Ryan EA, et al. Islet transplantation in seven patients with type 1 diabetes mellitus using a glucocorticoid-free immunosuppressive regimen. N Engl J Med 2000; 343: 230.
48. Kimmel SG, Ohbatake M, Kushida M, et al. Murine xenogeneic immune responses to the human testis: a presumed immune-privileged tissue. Transplantation 2000; 69: 1075.
49. Orth JM. Proliferation of Sertoli cells in fetal and postnatal rats: a quantitative autoradiography study. Anat Rec 1982; 203: 485.
50. Tran D, Meusy-Dessolle N, Josso N. Waning of antimüllerian activity: an early sign of Sertoli cell maturation in the developing pig. Biol Reprod 1981; 24: 923.
51. Franca LR, Silva VA Jr, Chiarini-Garcia H, Garcia SK, Debeljuk L. Cell proliferation and hormonal changes during postnatal development of the testis in the pig. Biol Reprod 2000; 63: 1629.
52. Cupp AS, Dufour JM, Kim G, Skinner MK, Kim KH. Actions of retinoids on embryonic and early postnatal testis development. Endocrinology 1999; 140: 2343.
53. Cupp AS, Kim G, Skinner MK. Expression and action of transforming growth factor beta (TGFbeta1, TGEbeta2, and TGFbeta3) during embryonic rat testis development. Biol Reprod 1999; 60: 1304.
54. Cupp AS, Kim GH, Skinner MK. Expression and action of neurotropin-3 and nerve growth factor in embryonic and early postnatal rat testis development. Biol Reprod 2000; 63: 1617.
55. Levine E, Cupp AS, Miyashiro L, Skinner MK. Role of transforming growth factor-alpha and the epidermal growth factor receptor in embryonic rat testis development. Biol Reprod 2000; 62: 477.
56. Kin T., Rajotte R.V., Dufour J. M., Korbutt G. S. Development of an Immunoprivileged Site to Prolong Islet Allograft Survival. Cell Transplantation 2002; 11: 547-552.
57. Gores P. F., Hayes D. H., Copeland M. J., Korbutt G. S., Halberstadt C., Kirkpatrick S.A., Rajotte R.V. Long-Term Survival of Intratesticular Porcine Islets In Nonimmunosuppressed Beagles. Transplantation 2003; 75(5): 613-618.
58. Kin T., Korbutt G. S., Rajotte R.V. Survival and Metabolic Function of Syngeneic Rat Islet Grafts Transplanted in the Omental Pouch. American Journal of Transplantation 2003; 3:281-285.
59. Binette T. M., Dufour J. M., Korbutt G. S. In Vitro Maturation of Neonatal Porcine Islets A Novel Model for the Study of Islet Development and Xenotransplantation. Ann. N Y Acad. Sci. 2001; 944: 47-61.
60. R.A. Valdes-Gomzalaez1, R.B. Elliot, L.M. Dorantes, L. Escobar, G.N. Garibay, E. Bracho, C. Macias, L. Silva1, L. Copeman, P. Valencia1, D.J.G. White. Porcine Islet Xenografts Can Survive And Function In Type 1 Diabetic Patients In The Presence Of Both Pre-Existing And Elicited Anti-Pig Antibodies. Abstract, the 19th International Transplant Society Congress, Miami, August, 2002.

## Claims

1. A pharmaceutical composition comprising Sertoli cells, myoid cells and a pharmaceutically acceptable carrier, for use in creating an immunologically privileged site in a mammal, wherein the ratio of said myoid cells to said Sertoli-cells is at least 5:95.

2. The pharmaceutical composition for use according to claim 1, wherein the ratio of said myoid cells versus said Sertoli cells is at least 25:75.

3. The pharmaceutical composition for use according to claim 1, wherein the ratio of said myoid cells versus said Sertoli cells is in the range of 5:95 to 65:35.

4. The pharmaceutical composition for use according to claim 1, wherein said Sertoli cells are obtained from a cell line.

5. The pharmaceutical composition for use according to claim 1, wherein said Sertoli cells and said myoid cells are obtained from the testis of a mammal.

6. The pharmaceutical composition for use according to claim 5, wherein said mammal is a pig.

7. The pharmaceutical composition for use according to claim 4, wherein said Sertoli cells are obtained from a human stem cell line.

8. The pharmaceutical composition for use according to claim 6, wherein said pig is 60 days old or younger.

9. The pharmaceutical composition for use according to claim 6, wherein said pig is 5 days old or younger.

10. The pharmaceutical composition for use according to any of claims 1-9, further comprising cells that produce insulin.

11. The pharmaceutical composition for use according to claim 10, wherein said cells that produce insulin are cells of pancreatic islets.

12. The pharmaceutical composition for use according to claim 11, wherein not more than 2 x 10⁶ Sertoli cells are used per 800 islets.

13. The pharmaceutical composition for use according to claim 1, provided in a device encapsulating the Sertoli cells and the myoid cells, wherein said device is suitable for implantation into a mammalian subject.

14. The pharmaceutical composition for use according to claim 1, wherein said Sertoli cells and said myoid cells are for administration subcutaneously into a site in the subject or intramuscularly.

15. The pharmaceutical composition for use according to claim 14, wherein said site is selected from the brain, the renal subcapsular space, the liver subcapsular space, the hepatic portal vein, the omental pouch, or the subcutaneous fascia.

16. The pharmaceutical composition for use according to claim 1, wherein said Sertoli cells and said myoid cells are for administration at a total amount ranging from 10⁵ to 10⁸ cells.

17. The pharmaceutical composition for use according to claim 16, wherein said total amount is 10⁷ to 10⁸ cells.

18. The pharmaceutical composition for use according to any previous claim, for the treatment of a human.

19. The pharmaceutical composition for use according to claim 1, for administration by implantation of a device encapsulating the cells or by transplantation.

20. The pharmaceutical composition for use according to claim 19, wherein said transplantation is an allograft or xenograft.

21. The pharmaceutical composition for use according to any previous claim, comprising aggregates obtained by co-culture of Sertoli and myoid cells under conditions to form Sertoli-myoid cell aggregates.

22. The pharmaceutical composition for use according to claim 1, wherein the Sertoli cells are engineered to produce a therapeutic protein.

23. Sertoli cells, myoid cells and a therapeutically effective amount of cells that produce insulin, for use in a method for the treatment of diabetes mellitus, wherein the ratio of said myoid cells to said Sertoli cells is at least 5:95.

24. The Sertoli cells, myoid cells and a therapeutically effective amount of cells that produce insulin for use according to claim 23, wherein the ratio of said myoid cells versus said Sertoli cells is at least 25:75.

25. The Sertoli cells, myoid cells and a therapeutically effective amount of cells that produce insulin for use according to claim 23, wherein the ratio of said myoid cells versus said Sertoli cells is in the range of 5:95 to 65:35.

26. The Sertoli cells, myoid cells and a therapeutically effective amount of cells that produce insulin for use according to claim 23, wherein said Sertoli cells are obtained from a cell line.

27. The Sertoli cells, myoid cells and a therapeutically effective amount of cells that produce insulin for use according to claim 23, wherein said Sertoli cells and said myoid cells are obtained from the testis of a mammal.

28. The Sertoli cells, myoid cells and a therapeutically effective amount of cells that produce insulin for use according to claim 27, wherein said mammal is a pig.

29. The Sertoli cells, myoid cells and a therapeutically effective amount of cells that produce insulin for use according to claim 23, wherein said Sertoli cells are obtained from a human stem cell line.

30. The Sertoli cells, myoid cells and a therapeutically effective amount of cells that produce insulin for use according to claim 28, wherein said pig is 60 days old or younger.

31. The Sertoli cells, myoid cells and a therapeutically effective amount of cells that produce insulin for use according to claim 28, wherein said pig is 5 days old or younger.

32. The Sertoli cells, myoid cells and a therapeutically effective amount of cells that produce insulin for use according to claim 23, for subcutaneous or intramuscular administration.

33. The Sertoli cells, myoid cells and a therapeutically effective amount of cells that produce insulin for use according to claim 32, for the administration to the brain, the renal subcapsular space, the liver subcapsular space, the hepatic portal vein, the omental pouch, or the subcutaneous fascia.

34. The Sertoli cells, myoid cells and a therapeutically effective amount of cells that produce insulin for use according to claim 33, wherein said Sertoli cells, said myoid cells and said cells that produce said insulin are administered at a total amount ranging from 10⁵ to 10⁸ cells.

35. The Sertoli cells, myoid cells and a therapeutically effective amount of cells that produce insulin for use according to claim 34, wherein said total amount is 10⁷ to 10⁸ cells.

36. The Sertoli cells, myoid cells and a therapeutically effective amount of cells that produce insulin for use according to claim 23, for treatment of a human.

37. The Sertoli cells, myoid cells and a therapeutically effective amount of cells that produce insulin for use according to claim 23, for administration by implantation of a device encapsulating the cells or by transplantation.

38. The Sertoli cells, myoid cells and a therapeutically effective amount of cells that produce insulin for use according to claim 37, wherein said transplantation is allograft or xenograft.

39. The Sertoli cells, myoid cells and a therapeutically effective amount of cells that produce insulin for use according to claim 23, wherein said cells that produce said biological factor are cells of pancreatic islets.

40. The Sertoli cells, myoid cells and a therapeutically effective amount of cells that produce insulin for use according to claim 39, wherein said Sertoli cells, said myoid cells and said cells of pancreatic islets are co-cultured to form Sertoli-myoid-islet cell aggregates prior to administration.

41. The Sertoli cells, myoid cells and a therapeutically effective amount of cells that produce insulin for use according to claim 39, wherein not more than 2 x 10⁶ Sertoli cells are used per 800 islets.

## Patentansprüche

1. Eine pharmazeutische Zusammensetzung umfassend Sertoli-Zellen, myoide Zellen und einen pharmazeutisch unbedenklichen Träger zur Verwendung beim Erstellen einer immunologisch privilegierten Stelle in einem Säuger, wobei das Verhältnis dieser myoiden Zellen zu diesen Sertoli-Zellen mindestens 5:95 beträgt.

2. Die pharmazeutische Zusammensetzung zur Verwendung nach Anspruch 1, wobei das Verhältnis dieser myoiden Zellen gegenüber diesen Sertoli-Zellen mindestens 25:75 beträgt.

3. Die pharmazeutische Zusammensetzung zur Verwendung nach Anspruch 1, wobei das Verhältnis dieser myoiden Zellen gegenüber diesen Sertoli-Zellen im Bereich 5:95 bis 65:35 liegt.

4. Die pharmazeutische Zusammensetzung zur Verwendung nach Anspruch 1, wobei diese Sertoli-Zellen aus einer Zelllinie gewonnen werden.

5. Die pharmazeutische Zusammensetzung zur Verwendung nach Anspruch 1, wobei diese Sertoli-Zellen und diese myoiden Zellen aus dem Hoden eines Säugers gewonnen werden.

6. Die pharmazeutische Zusammensetzung zur Verwendung nach Anspruch 5, wobei dieser Säuger ein Schwein ist.

7. Die pharmazeutische Zusammensetzung zur Verwendung nach Anspruch 4, wobei diese Sertoli-Zellen aus einer menschlichen Stammzellenlinie gewonnen werden.

8. Die pharmazeutische Zusammensetzung zur Verwendung nach Anspruch 6, wobei dieses Schwein 60 Tage oder jünger ist.

9. Die pharmazeutische Zusammensetzung zur Verwendung nach Anspruch 6, wobei dieses Schwein 5 Tage oder jünger ist.

10. Die pharmazeutische Zusammensetzung zur Verwendung nach einem der Ansprüche 1-9, ferner umfassend insulinproduzierende Zellen.

11. Die pharmazeutische Zusammensetzung zur Verwendung nach Anspruch 10, wobei es sich bei diesen insulinproduzierenden Zellen um Zellen von Langerhans-Inseln handelt.

12. Die pharmazeutische Zusammensetzung zur Verwendung nach Anspruch 11, wobei nicht mehr als 2 x 10⁶ Sertoli-Zellen pro 800 Inseln verwendet werden.

13. Die pharmazeutische Zusammensetzung zur Verwendung nach Anspruch 1, bereitgestellt in einer die Sertoli-Zellen und die myoiden Zellen umschließenden Vorrichtung, wobei diese Vorrichtung für die Implantation in ein Säugersubjekt geeignet ist.

14. Die pharmazeutische Zusammensetzung zur Verwendung nach Anspruch 1, wobei diese Sertoli-Zellen und diese myoiden Zellen zur Verabreichung subkutan an einer Stelle in dem Subjekt oder intramuskulär vorgesehen sind.

15. Die pharmazeutische Zusammensetzung zur Verwendung nach Anspruch 14, wobei diese Stelle aus dem Gehirn, dem subkapsulären Raum der Niere, dem subkapsulären Raum der Leber, der hepatischen Pfortader, der Netztasche oder der subkutanen Faszie ausgewählt wird.

16. Die pharmazeutische Zusammensetzung zur Verwendung nach Anspruch 1, wobei diese Sertoli-Zellen und diese myoiden Zellen zur Verabreichung in einer Gesamtmenge im Bereich von 10⁵ bis 10⁸ Zellen vorgesehen sind.

17. Die pharmazeutische Zusammensetzung zur Verwendung nach Anspruch 16, wobei diese Gesamtmenge zwischen 10⁷ und 10⁸ Zellen liegt.

18. Die pharmazeutische Zusammensetzung zur Verwendung nach einem der vorstehenden Ansprüche für die Behandlung eines Menschen.

19. Die pharmazeutische Zusammensetzung zur Verwendung nach Anspruch 1 zur Verabreichung durch Implantation einer die Zellen umschließenden Vorrichtung oder durch Transplantation.

20. Die pharmazeutische Zusammensetzung zur Verwendung nach Anspruch 19, wobei diese Transplantation ein Allotransplantat oder ein Xenotransplantat ist.

21. Die pharmazeutische Zusammensetzung zur Verwendung nach einem der vorstehenden Ansprüche, umfassend durch Co-Kultur aus Sertoli- und myoiden Zellen gewonnene Aggregate unter Bedingungen zur Bildung von Zellaggregaten aus Sertoli-Zellen und myoiden Zellen.

22. Die pharmazeutische Zusammensetzung zur Verwendung nach Anspruch 1, wobei die Sertoli-Zellen so hergestellt sind, um ein therapeutisches Protein zu produzieren.

23. Sertoli-Zellen, myoide Zellen und eine therapeutisch wirksame Menge von insulinproduzierenden Zellen zur Verwendung in einem Verfahren für die Behandlung von Diabetes mellitus, wobei das Verhältnis dieser myoiden Zellen zu diesen Sertoli-Zellen mindestens 5:95 beträgt.

24. Die Sertoli-Zellen, myoide Zellen und eine therapeutisch wirksame Menge von insulinproduzierenden Zellen zur Verwendung nach Anspruch 23, wobei das Verhältnis dieser myoiden Zellen gegenüber diesen Sertoli-Zellen mindestens 25:75 beträgt.

25. Die Sertoli-Zellen, myoide Zellen und eine therapeutisch wirksame Menge von insulinproduzierenden Zellen zur Verwendung nach Anspruch 23, wobei das Verhältnis dieser myoiden Zellen gegenüber diesen Sertoli-Zellen im Bereich von 5:95 bis 65:35 liegt.

26. Die Sertoli-Zellen, myoide Zellen und eine therapeutisch wirksame Menge von insulinproduzierenden Zellen zur Verwendung nach Anspruch 23, wobei diese Sertoli-Zellen aus einer Zelllinie gewonnen werden.

27. Die Sertoli-Zellen, myoide Zellen und eine therapeutisch wirksame Menge von insulinproduzierenden Zellen zur Verwendung nach Anspruch 23, wobei diese Sertoli-Zellen und diese myoiden Zellen aus dem Hoden eines Säugers gewonnen werden.

28. Die Sertoli-Zellen, myoide Zellen und eine therapeutisch wirksame Menge von insulinproduzierenden Zellen zur Verwendung nach Anspruch 27, wobei dieser Säuger ein Schwein ist.

29. Die Sertoli-Zellen, myoide Zellen und eine therapeutisch wirksame Menge von insulinproduzierenden Zellen zur Verwendung nach Anspruch 23, wobei diese Sertoli-Zellen aus einer menschlichen Stammzellenlinie gewonnen werden.

30. Die Sertoli-Zellen, myoide Zellen und eine therapeutisch wirksame Menge von insulinproduzierenden Zellen zur Verwendung nach Anspruch 28, wobei dieses Schwein 60 Tage alt oder jünger ist.

31. Die Sertoli-Zellen, myoide Zellen und eine therapeutisch wirksame Menge von insulinproduzierenden Zellen zur Verwendung nach Anspruch 28, wobei dieses Schwein 5 Tage alt oder jünger ist.

32. Die Sertoli-Zellen, myoide Zellen und eine therapeutisch wirksame Menge von insulinproduzierenden Zellen zur Verwendung nach Anspruch 23 zur subkutanen oder intramuskulären Verabreichung.

33. Die Sertoli-Zellen, myoide Zellen und eine therapeutisch wirksame Menge von insulinproduzierenden Zellen zur Verwendung nach Anspruch 32 zur Verabreichung an das Gehirn, den subkapsulären Raum der Niere, den subkapsulären Raum der Leber, die hepatische Pfortader, die Netztasche oder die subkutane Faszie.

34. Die Sertoli-Zellen, myoide Zellen und eine therapeutisch wirksame Menge von insulinproduzierenden Zellen zur Verwendung nach Anspruch 33, wobei diese Sertoli-Zellen, diese myoiden Zellen und diese insulinproduzierenden Zellen in einer Gesamtmenge im Bereich von 10⁵ bis 10⁸ Zellen verabreicht werden.

35. Die Sertoli-Zellen, myoide Zellen und eine therapeutisch wirksame Menge von insulinproduzierenden Zellen zur Verwendung nach Anspruch 34, wobei diese Gesamtmenge zwischen 10⁷ und 10⁸ Zellen liegt.

36. Die Sertoli-Zellen, myoide Zellen und eine therapeutisch wirksame Menge von insulinproduzierenden Zellen zur Verwendung nach Anspruch 23 zur Behandlung eines Menschen.

37. Die Sertoli-Zellen, myoide Zellen und eine therapeutisch wirksame Menge von insulinproduzierenden Zellen zur Verwendung nach Anspruch 23 zur Verabreichung durch Implantation einer die Zellen umschließenden Vorrichtung oder durch Transplantation.

38. Die Sertoli-Zellen, myoide Zellen und eine therapeutisch wirksame Menge von insulinproduzierenden Zellen zur Verwendung nach Anspruch 37, wobei diese Transplantation ein Allotransplantat oder ein Xenotransplantat ist.

39. Die Sertoli-Zellen, myoide Zellen und eine therapeutisch wirksame Menge von insulinproduzierenden Zellen zur Verwendung nach Anspruch 23, wobei diese diesen biologischen Faktor produzierenden Zellen Zellen von Langerhans-Inseln sind.

40. Die Sertoli-Zellen, myoide Zellen und eine therapeutisch wirksame Menge von insulinproduzierenden Zellen zur Verwendung nach Anspruch 39, wobei diese Sertoli-Zellen, diese myoiden Zellen und diese Zellen von Langerhans-Inseln co-kultiviert sind, um vor Verabreichung Zellaggregate aus Inseln von Sertoli-Zellen und myoiden Zellen zu bilden.

41. Die Sertoli-Zellen, myoide Zellen und eine therapeutisch wirksame Menge von insulinproduzierenden Zellen zur Verwendung nach Anspruch 39, wobei nicht mehr als 2 x 10⁶ Sertoli-Zellen pro 800 Inseln verwendet werden.

## Revendications

1. Composition pharmaceutique comprenant des cellules de Sertoli, des cellules myoïdes et un excipient pharmaceutiquement acceptable, destinée à être utilisée pour créer un site privilégié immunologiquement chez un mammifère, le rapport desdites cellules myoïdes sur lesdites cellules de Sertoli étant au moins égal à 5:95.

2. Composition pharmaceutique destinée à être utilisée selon la revendication 1, ledit rapport desdites cellules myoïdes sur lesdites cellules de Sertoli étant au moins égal à 25:75.

3. Composition pharmaceutique destinée à être utilisée selon la revendication 1, ledit rapport desdites cellules myoïdes sur lesdites cellules de Sertoli étant compris dans la plage allant de 5:95 à 65:35.

4. Composition pharmaceutique destinée à être utilisée selon la revendication 1, lesdites cellules de Sertoli étant obtenues à partir d'une lignée cellulaire.

5. Composition pharmaceutique destinée à être utilisée selon la revendication 1, lesdites cellules de Sertoli et lesdites cellules myoïdes étant obtenues à partir des testicules d'un mammifère.

6. Composition pharmaceutique destinée à être utilisée selon la revendication 5, ledit mammifère étant un porc.

7. Composition pharmaceutique destinée à être utilisée selon la revendication 4, lesdites cellules de Sertoli étant obtenues à partir d'une lignée de cellules souches humaines.

8. Composition pharmaceutique destinée à être utilisée selon la revendication 6, ledit porc étant âgé de 60 jours ou moins.

9. Composition pharmaceutique destinée à être utilisée selon la revendication 6, ledit porc étant âgé de 5 jours ou moins.

10. Composition pharmaceutique destinée à être utilisée selon l'une quelconque des revendications 1 à 9 comprenant en outre des cellules qui produisent de l'insuline.

11. Composition pharmaceutique destinée à être utilisée selon la revendication 10, lesdites cellules qui produisent de l'insuline étant des cellules d'îlots pancréatiques.

12. Composition pharmaceutique destinée à être utilisée selon la revendication 11, pas plus de 2 x 10⁶ cellules de Sertoli étant utilisées pour 800 îlots.

13. Composition pharmaceutique destinée à être utilisée selon la revendication 1 pourvue dans un dispositif encapsulant les cellules de Sertoli et les cellules myoïdes, ledit dispositif étant adapté à une implantation dans un sujet mammifère.

14. Composition pharmaceutique destinée à être utilisée selon la revendication 1, lesdites cellules de Sertoli et lesdites cellules myoïdes étant destinées à une administration par voie sous-cutanée dans un site chez le sujet ou par voie intramusculaire.

15. Composition pharmaceutique destinée à être utilisée selon la revendication 14, ledit site étant choisi parmi le cerveau, l'espace sous-capsulaire rénal, l'espace sous-capsulaire hépatique, la veine porte hépatique, la poche épiploïque ou le fascia sous-cutané.

16. Composition pharmaceutique destinée à être utilisée selon la revendication 1, lesdites cellules de Sertoli et lesdites cellules myoïdes étant destinées à l'administration d'une quantité totale allant de 10⁵ à 10⁸ cellules.

17. Composition pharmaceutique destinée à être utilisée selon la revendication 16, ladite quantité totale représentant 10⁷ à 10⁸ cellules.

18. Composition pharmaceutique destinée à être utilisée selon l'une quelconque des revendications précédentes pour le traitement d'un humain.

19. Composition pharmaceutique destinée à être utilisée selon la revendication 1 pour administration par implantation d'un dispositif encapsulant les cellules ou par transplantation.

20. Composition pharmaceutique destinée à être utilisée selon la revendication 19, ladite transplantation étant une allogreffe ou une xénogreffe.

21. Composition pharmaceutique destinée à être utilisée selon l'une quelconque des revendications précédentes comprenant des agrégats obtenus par co-culture de cellules Sertoli et de cellules myoïdes dans des conditions permettant de produire des agrégats de cellules de Sertoli et de cellules myoïdes.

22. Composition pharmaceutique destinée à être utilisée selon la revendication 1, lesdites cellules de Sertoli étant conçues pour produire une protéine thérapeutique.

23. Cellules de Sertoli, cellules myoïdes et quantité thérapeutiquement efficace de cellules qui produisent de l'insuline, destinées à être utilisées dans un mode de traitement du diabète sucré, ledit rapport desdites cellules myoïdes sur lesdites cellules de Sertoli étant au moins égal à 5:95.

24. Cellules de Sertoli, cellules myoïdes et quantité thérapeutiquement efficace de cellules qui produisent de l'insuline destinées à être utilisées selon la revendication 23, ledit rapport desdites cellules myoïdes sur lesdites cellules de Sertoli étant au moins égal à 25:75.

25. Cellules de Sertoli, cellules myoïdes et quantité thérapeutiquement efficace de cellules qui produisent de l'insuline destinées à être utilisée selon la revendication 23, ledit rapport desdites cellules myoïdes sur lesdites cellules de Sertoli étant compris dans la plage allant de 5:95 à 65:35.

26. Cellules de Sertoli, cellules myoïdes et quantité thérapeutiquement efficace de cellules qui produisent de l'insuline destinées à être utilisées selon la revendication 23, lesdites cellules de Sertoli étant obtenues à partir d'une lignée cellulaire.

27. Cellules Sertoli, cellules myoïdes et quantité thérapeutiquement efficace de cellules qui produisent de l'insuline destinées à être utilisées selon la revendication 23, lesdites cellules de Sertoli et lesdites cellules myoïdes étant obtenues à partir des testicules d'un mammifère.

28. Cellules de Sertoli, cellules myoïdes et quantité thérapeutiquement efficace de cellules qui produisent de l'insuline destinées à être utilisées selon la revendication 27, ledit mammifère étant un porc.

29. Cellules de Sertoli, cellules myoïdes et quantité thérapeutiquement efficace de cellules qui produisent de l'insuline destinées à être utilisées selon la revendication 23, lesdites cellules de Sertoli étant obtenues à partir d'une lignée de cellules souches humaines.

30. Cellules de Sertoli, cellules myoïdes et quantité thérapeutiquement efficace de cellules qui produisent de l'insuline destinées à être utilisées selon la revendication 28, ledit porc étant âgé de 60 jours ou moins.

31. Cellules de Sertoli, cellules myoïdes et quantité thérapeutiquement efficace de cellules qui produisent de l'insuline destinées à être utilisées selon la revendication 28, ledit porc étant âgé de 5 jours ou moins.

32. Cellules de Sertoli, cellules myoïdes et quantité thérapeutiquement efficace de cellules qui produisent de l'insuline destinées à être utilisées selon la revendication 23 pour administration sous-cutanée ou intramusculaire.

33. Cellules de Sertoli, cellules myoïdes et quantité thérapeutiquement efficace de cellules qui produisent de l'insuline destinées à être utilisées selon la revendication 32 pour administration au cerveau, à l'espace sous-capsulaire rénal, à l'espace sous-capsulaire hépatique, à la veine porte hépatique, à la poche épiploïque ou au fascia sous-cutané.

34. Cellules de Sertoli, cellules myoïdes et quantité thérapeutiquement efficace de cellules qui produisent de l'insuline destinées à être utilisées selon la revendication 33, lesdites cellules de Sertoli, lesdites cellules myoïdes et lesdites cellules qui produisent ladite insuline étant administrées en une quantité totale allant de 10⁵ à 10⁸ cellules.

35. Cellules de Sertoli, cellules myoïdes et quantité thérapeutiquement efficace de cellules qui produisent de l'insuline destinées à être utilisées selon la revendication 34, ladite quantité totale représentant 10⁷ à 10⁸ cellules.

36. Cellules de Sertoli, cellules myoïdes et quantité thérapeutiquement efficace de cellules qui produisent de l'insuline destinées à être utilisées selon la revendication 23 pour traitement d'un humain.

37. Cellules de Sertoli, cellules myoïdes et quantité thérapeutiquement efficace de cellules qui produisent de l'insuline destinées à être utilisées selon la revendication 23 pour administration par implantation d'un dispositif encapsulant les cellules ou par transplantation.

38. Cellules de Sertoli, cellules myoïdes et quantité thérapeutiquement efficace de cellules qui produisent de l'insuline destinées à être utilisées selon la revendication 27, ladite transplantation étant une allogreffe ou une xénogreffe.

39. Cellules de Sertoli, cellules myoïdes et quantité thérapeutiquement efficace de cellules qui produisent de l'insuline destinées à être utilisées selon la revendication 23, lesdites cellules qui produisent ledit facteur biologique étant des cellules d'îlots pancréatiques.

40. Cellules de Sertoli, cellules myoïdes et quantité thérapeutiquement efficace de cellules qui produisent de l'insuline destinées à être utilisées selon la revendication 39, lesdites cellules de Sertoli, lesdites cellules myoïdes et lesdites cellules d'îlots pancréatiques étant co-cultivées pour produire des agrégats cellules de Sertoli-cellules myoïdes-cellules d'îlots avant administration.

41. Cellules de Sertoli, cellules myoïdes et quantité thérapeutiquement efficace de cellules qui produisent de l'insuline destinées à être utilisées selon la revendication 39, pas plus de 2 x 10⁶ cellules de Sertoli étant utilisées pour 800 îlots.
